# EUROPEAN PATENT SPECIFICATION

(11) **EP 2 773 402 B1**
(45) Date of publication and mention of the grant of the patent: **30.12.2015**
(21) Application number: 12775698.9
(22) Date of filing: 25.10.2012
(51) Int. Cl.: A61M 5/32, A61B 17/34, B21G 1/00

(54) **PROCESS FOR SHAPING A NEEDLE CANNULA**
VERFAHREN ZUM FORMEN EINER NADELKANÜLE
PROCÉDÉ DE MISE EN FORME D'UNE CANULE À AIGUILLE

(30) Priority: 03.11.2011 EP 11187689; 07.11.2011 US 201161556421 P
(43) Date of publication of application: 10.09.2014
(73) Proprietor: Novo Nordisk A/S, 2880 Bagsværd (DK)
(72) Inventor: SØRENSEN, Dan Nørtoft, 2880 Bagsværd (DK); ØHLENSCHLÆGER, Rasmus, 2880 Bagsværd (DK)
(86) International application number: PCT/EP2012/071111
(87) International publication number: WO 2013/064414

(56) References cited:
- EP-A1- 0 445 820
- WO-A1-2011/033102
- US-A- 3 192 084
- US-A- 4 335 718
- US-A- 4 587 202
- US-A- 5 693 454
- US-A1- 2005 044 922

## Description

### THE TECHNICAL FIELD OF THE INVENTION:

The invention relates to a method of manufacturing a needle cannula having improved flow properties and a needle cannula obtained by the method.

### DESCRIPTION OF RELATED ART:

Needle assemblies are commonly used to either inject substances into or extract substances out of human or animal bodies. Such needle assemblies are typically disposable and are discarded after only one use.

When producing needle assemblies a needle cannula which is typically drawn from stainless steel is attached to a hub generally moulded from a suitable polymer. The hub usually carries means for attaching the needle assembly to an injection device. The needle cannula is typically mounted to the hub such that a liquid communication patch can be established between the injection device, through the needle assembly and into the body of the user.

The needle cannula is normally made from a stainless-steel tube drawn through progressively smaller dies to make the needle the wanted diameter. The ratio between the outside diameter and the inside diameter is a result of the wall thickness of the stainless steel tube drawn and the diameter to which it is drawn. The specific dimensions used for a cylindrical needle cannula are given in ISO 9626.

Some drugs, such as insulin are self-administered, and the typical diabetes person will require subcutaneous injections of insulin several times during the course of the day. Recent studies have indicated that people who inject themselves experience less pain when using a thin needle i.e. a needle cannula having a little outside diameter. In order to reduce the discomfort of having to inject oneself several times a day, injection needles with a very thin needle cannula are very popular among people suffering from diabetes.

The outside diameter of a needle cannula is indicated by a "G" followed by a gauge number, which gauge number increases with thinner needles. At the present, the most commonly used injection needles among people suffering from diabetes are G30 or G31. Thus the outside diameter of a G 30 is according to ISO 9626 approximately 0,3 millimetres and of a G 31 approximately 0,25 millimetres. However, injection needles as thin as G32 are available today. A G32 needle has an outside diameter of approximately 0,23 mm.

As needle cannulae are drawn from a cylindrical tube, the inside diameter decreases as the outside diameter decreases. A major problem with needle cannulae having a very thin outside diameter is that the inside diameter is also very small which requires a relatively large force to press the liquid drug through the lumen and into the user.

Fully developed laminar flows in pipes can be expressed by *Pouseuilles formula.* The flow of a liquid medicament through a thin metal needle cannula can in many aspects be assumed to also behave according to this formula. According to this formula, as explained in US 5,951,528, it is preferred to have as large an inside diameter as possible and as short a length of the needle cannula as possible in order to maximise the flow through the needle cannula since the fourth ratio of the inside diameter is divided with the length in the formula. When discussing a needle cannula tapered on the inside, the sum of all contributions determine the total flow resistance, and the largest inside diameter must preferably be on the longest contributing part of the total length in order to increase the flow. Thus, the length of the needle cannula having the narrow tubing should be minimized.

A method of producing a needle cannula having a tapered inside is disclosed in WO 2011/033102. In this method material is removed from the inside wall of the lumen on a restricted part of the total length of the needle cannula by applying a liquid etchant to a limited part of the lumen. In this method, a pressure is build up in the liquid etchant by using centrifugal force. Once an adequate pressure is build up, the needle cannula and the liquid etchant are moved relatively to each other a distance determined by the length from which wall material is desired to be removed. However, since the process require both a centrifugal force and a relative motion, the process is rather difficult to carry out.

Document US 3 192 084 A describes a method of manufacturing a needle, said method using a pressurized metal etching liquid.

### DESCRIPTION OF THE INVENTION:

Having regard to the above identified prior art, it is an object of the present invention to provide a simple, cheap and reliable method for manufacturing a needle cannula with improved flow characteristic combining a small outside diameter with a satisfactory resistance against breakage when bended.

Further, it is an object to provide a method by which the interior material of a needle cannula can be chemically removed thereby increasing the flow through the needle cannula and especially a method by which material is removed from only a part of the axial length of the needle cannula thereby improving the flow through the needle cannula but maintaining the resistance against breakage.

In a first aspect, the present invention relates to a method involving the following steps preferably but not necessarily performed in the below order:
(i) providing a plurality of metallic needle cannulae having a first end and a second end and an oblong inside lumen there between,
(ii) providing a metal etching liquid surfacing a gas,
(iii) establishing contact between the metal etching liquid and one end of each needle cannula in the plurality,
(iv) sealing off the opposite end of each needle cannula in the plurality,
(v) transferring the metal etching liquid into a part of the oblong inside lumen of the metallic needle cannula, by applying pressure to the gas surfacing the metal etching liquid.

Once one end of the individual needle cannula is submerged in the liquid and the opposite end is sealed off a volume of gas is trapped inside the lumen of the needle cannula. Once the pressure of the gas surfacing the metal etching liquid is raised above the pressure of the entrapped gas, the pressure of the entrapped gas will increase and the volume of the entrapped gas will decrease, or in other words, the liquid will flow further into the lumen of the needle cannula.

Since the entrapped gas cannot escape due to the seal there will always be a volume inside the lumen of the needle cannula into which the metal etching liquid will never reach. Due to this a part of the lumen will never be exposed to the metal etching liquid. As a result the inside lumen of the etched needle cannula will have a first part which has the original diameter and a second part that has a larger diameter as material has been etched away. The first and the second area will be connected by a transition area which could be conical due to the surface of the metal etching liquid inside the lumen.

The level inside the lumen to which the metal etching liquid rises can be calculated using the ideal gas equation. As an example, if the pressure of the gas surfacing the metal etching liquid increases from 1 bar to 5 bar, the volume of the entrapped gas falls to a fifth part of the original volume following the ideal gas equation. For a constant-diameter needle cannula this means that the etchant level rises to a level four/fifth part into the needle cannula.

Once the above method has been carried out, the metal etching liquid is removed from the lumen by releasing the pressure and removing the seal which will cause the metal etching liquid to flow out of the lumen.

In one embodiment, the metal etching liquid is contained in a bath and transferred to the inside of the lumen by applying a pressure to the gas surfacing the surface of the liquid in the bath. When one end of the needle cannula is submerged into the bath and the free end is sealed of, a pressure applied to the surface of the bath will cause the level of the metal etching liquid to rise inside the lumen as the entrapped gas is compressed.

The process is in a further embodiment carried out inside a pressure chamber which pressure chamber preferably is divided into two different gas-chambers separated from each other by a gas tight barrier. The two gas-chambers are connected through the lumen of the needle cannula and different gas pressures can be applied to the two gas-chambers in order to control the flow of the metal etching liquid as explained in more details later. The barrier need not be 100 percent gas tight as long as it is possible to control the gas pressures of the two gas-chambers.

Further, the steps of the method can be performed any number of times until the inside lumen is shaped as wanted. In a situation of use, the individual needle cannula is mounted in a hub which hub secures the needle cannula to an injection device. The point in which the needle cannula is attached to the hub should preferably be the area in which the needle cannula has the largest wall thickness and thereby the greatest resistance to bending. The level of the metal etching liquid inside the lumen should therefore be maintained during the process in a position facilitating the above. If e.g. 4 mm of the needle cannula is to be situated above the hub i.e. the injection length of the injection needle is 4 mm, the carved out portion of the lumen should preferably not extend into the distal 5-6 mm of the length of needle such that 1-2 mm of the axial length having the largest wall thickness is located at the point of attachment.

In a further development of the invention, the proximal end of the needle cannulae with etchant contained inside the lumen the needle cannulae is moved to a second bath containing a non-etching liquid such as water and the pressure of the surfacing gas is increased. As a result of this the etchant will be moved further into the lumen such that an area of the inside wall provided at an axial distance from the proximal end of the needle cannulae will be etched away without etching the most proximal end of the needle cannula.

In a second aspect the present invention relates to a needle cannula obtained by the method described above. Such needle cannula has flow properties which are superior when compared to the needle cannula initially used in the process due to the carved out portion of the lumen. Following the process, the outside distal tip of the needle cannula is preferably also etched as described in details in EP 1,331,958. In an example if e.g. a G32 needle cannula is exposed to the process, the flow properties of a G31 needle can be obtained. Following this process, the distal tip can be etched to the outer diameter of a G33 needle cannula. The resulting needle cannula having the outer appearance of a G33 needle cannula at the tip however with the flow properties of a G31 needle cannula and a resistance to bending as the G32 needle cannula.

For the method a number of different metal etching liquids are usable as e.g. described in "Fabrication of microchannels on stainless steel by wet chemical etching" by P. N. Rao and D. Kunzru published in Journal of Micromechanics and Microengineering 17, October 2007. In order to increase the removal of material, polishing grains can be added to the metal etching liquid.

### DEFINITIONS:

According to ISO 9626, the dimensions for the gauges G30 to G33 are as below:

| Guage Size | Designated Metric Size (mm) | Range of Outside Diameter (mm) | Min. inside Diameter (mm) |
|---|---|---|---|
| G33 | 0,2 | 0,203 - 0,216 | 0,089 |
| G32 | 0,23 | 0,229 - 0,241 | 0,089 |
| G31 | 0,25 | 0,245 - 0,267 | 0,114 |
| G30 | 0,3 | 0,298 - 0,320 | 0,133 |

An "injection pen" is typically an injection apparatus i.e. any apparatus for performing an injection of a drug having an oblong or elongated shape somewhat like a pen for writing. Although such pens usually have a tubular cross-section, they could easily have a different cross-section such as triangular, rectangular or square or any variation around these geometries.

As used herein, the term "drug" is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

Correspondingly, the term "subcutaneous" injection is meant to encompass any method of transcutaneous delivery to a subject.

The term "Needle Cannula" is used to describe the actual conduit performing the penetration of the skin during injection. A needle cannula is usually made from a metallic material such as stainless steel and connected to a hub to form a complete injection needle also often referred to as a "needle assembly". A needle cannula could however also be made from a polymeric material or a glass material. The needle cannula is mounted in a "hub", which also carries the connecting means for connecting the injection needle to an injection apparatus and is usually moulded from a suitable thermoplastic material. The "connection means" could as examples be a luer coupling, a bayonet coupling, a threaded connection or any combination thereof e.g. a combination as described in EP 1,536,854.

The term "Needle unit" is often used to describe the finished unit as delivered to a consumer, such unit usually consist of a sealed container housing one single needle assembly. Such needle unit or in fact the needle container of a unit usually has a closed distal end and an open proximal end which is sealed by a removable seal. The interior of such container is usually sterile such that the needle assembly is ready-to-use. Needle units special designed for pen injections systems are defined in ISO standard No. 11608 and is often referred to as "pen needles".

"Cartridge" is the term used to describe the container containing the drug. Cartridges are usually made from glass but could also be made from any suitable polymer e.g. by moulding or extrusion. A cartridge or ampoule is preferably sealed at one end by a pierceable membrane which can be pierced e.g. by an injection needle. The opposite end is closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. As an alternative to a cartridge, a flexible reservoir could be used.

All references, including publications, patent applications, and patents, cited herein are incorporated by reference in their entirety and to the same extent as if each reference were individually and specifically indicated to be incorporated by reference and were set forth in its entirety herein.
All headings and sub-headings are used herein for convenience only and should not be constructed as limiting the invention in any way.
The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.
This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1: show a schematic view of the set-up of the process according to the present invention.
- Figure 2-5: show the various steps of the process.
- Figure 6: show a schematic view of the process before and after the etching.
- Figure 7: show an alternative embodiment of the process.

The figures are schematic and simplified for clarity, and they just show details, which are essential to the understanding of the invention, while other details are left out. Throughout, the same reference numerals are used for identical or corresponding parts.

### DETAILED DESCRIPTION OF AN EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as there relative dimensions are intended to serve illustrative purposes only.

In that context it may be convenient to define that the term "distal end" in the appended figures is meant to refer to the end of the needle cannula penetrating the patient whereas the term "proximal end" is meant to refer to the opposite end of the needle cannula.

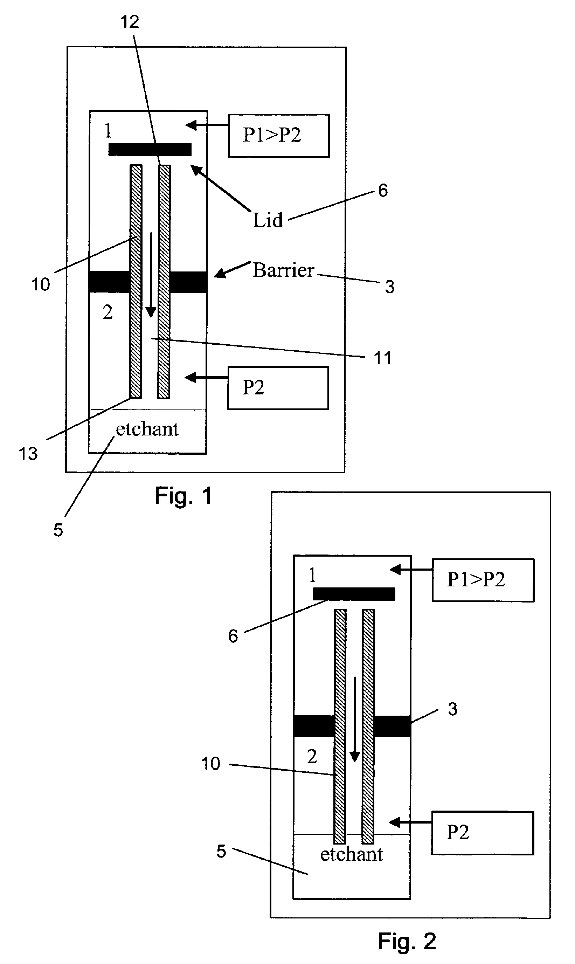

Figure 1 to figure 5 discloses the process of shaping the lumen 11 of a needle cannula 10.

The needle cannula 10 or preferably a plurality of needle cannulae 10 is placed inside a compartment comprising two separate gas-chambers 1, 2. The needle cannula 10 has an internal lumen 11 connecting the first gas-chamber 1 with the second gas-chamber 2 through an otherwise gas tight barrier 3. The distal end 12 of the needle cannula 10 is located in the first gas-chamber 1 and the proximal end 13 is located in the second gas-chamber 2. In the bottom of the second gas-chamber 2 a metal etching liquid 5 is provided preferably in the form of a bath. In the upper part of the first gas-chamber 1, a lid 6 is provided which can be pressed against the distal end 12 and seal off the lumen 11 of the needle cannula 10.

The further cycles of the process will hereafter be described in relation to the figures 1 to figure 5. In figure 1 the needle cannula 10 is free of the etchant 5 and the lid 6 is open. A pressure P1 is applied to the first gas-chamber 1. This pressure P1 is slightly higher than a pressure P2 of the second gas-chamber 2 such that gas flow from the first gas-chamber 1 and into the second gas-chamber 2 through the lumen 11 of the needle cannula 10.

In figure 2, the needle cannula 10 is submerged into the etchant 5 such that the opening of the lumen 11 at the proximal end 13 of the needle cannula 10 is kept under the level of the etchant 5. The gas pressures P1 and P2 are kept at there previous level which prevents the etchant 5 from entering into the lumen 11 as the pressure P1 is slightly higher than the pressure P2 of the second gas-chamber 2.

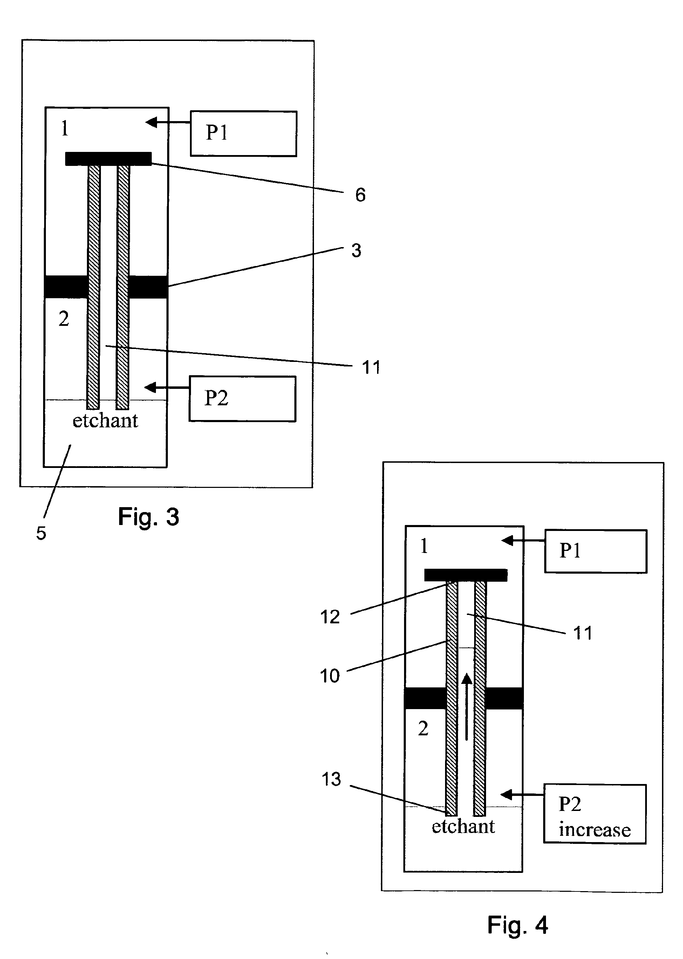

In figure 3, the lid 6 is closed whereby the gas will stop flowing through the lumen 11. The pressure of the gas trapped inside the lumen 11 is now that of gas-chamber 1 i.e. the pressure is P1. However, as P1 is slightly higher than P2, gas will continue to flow from inside the lumen 11 through the etchant 5 and into the gas-chamber 2 until the pressure inside the lumen settles at P2.

In figure 4, the pressure P2 is increased whereby the etchant 5 is forced to rise inside the lumen 11 of the needle cannula 10. The gas trapped in the lumen 11 is thus compressed and the level of the etchant 5 in the lumen 11 settles at a new level when the pressure of the trapped gas equals that of P2. The distance the etchant 5 travels inside the lumen 11 is thus determined by the pressure P2. As an example, if the pressure increases from 1 bar to 5 bar, the volume of the entrapped gas falls to a fifth part of the original volume following the ideal gas equation. For a constant-diameter needle cannulae 10 this means that the etchant 5 level rises to a level four/fifth part into the needle cannulae 10.

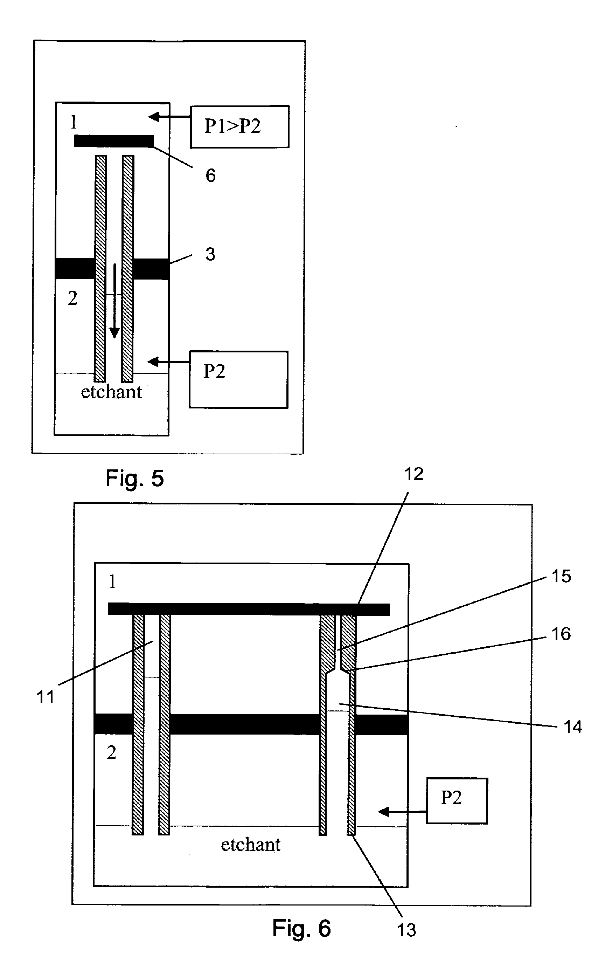

The etchant 5 inside the lumen 11 will etch off material from the sidewall of the lumen 11 thereby shaping the inside lumen 11 as depictured in the right part of figure 6. The shape of the lumen 11 will be such that material has been removed from the exposed part 14 of the lumen 11 which has been in contact with the etchant 5, whereas the non-exposed part 15 of the lumen 11 possesses the initial inside diameter of the needle cannula 10. The area 16 connecting the exposed part 14 and the non-exposed part 15 will tend to have a slightly tapered surface due the contact with the etchant 5.

When the needle cannula 10 is later embedded in a hub for use as an injection needle, it is preferred that the point of contact with the hub is where the needle cannula 10 has its thickest wall thickness. The connecting area 16 should therefore be located a distance from the distal tip of needle cannula 10 being slightly larger than the injection length of the needle cannula. If the injection length is e.g. 4 mm, the connecting area should preferably be located approximately 5 mm from the tip of the needle cannula.

In figure 5, the gas pressure P2 is released and returned to the initial pressure described in connection with figure 1. Further, the lid 6 is opened. The gas pressure P1 of the first gas-chamber now being above the pressure P2 of the second gas-chamber will press the etchant 5 proximally back to its initial level.

The cycles of the process can shortly be described as:
1) Setting the pressure P1 above P2 (fig. 1).
2) Lowering the needle cannula into the etchant (fig. 2).
3) Closing the lid (fig. 3).
4) Increasing the pressure P2 (fig. 4).
5) Holding the pressure P2 for a specific time period.
6) Releasing the pressure P2 and opening the lid (fig. 5).

These cycles can be repeated over and over again as many times as needed to obtain the desired shape of the inside lumen 11. When repeating the steps, individual steps can be excluded if wanted.

When carrying out the process a plurality of needle cannulae 10 can be bundled together either by chemical means or by mechanical means. However, this will create a number of leaks between the individual needle cannulae 10 in the bundle. When the pressure P1 is higher than the pressure P2 a gas flow between the individual needle cannulae 10 flow from gas-chamber 1 to gas-chamber 2 thus keeping any etchant 5 away from the outside surface of the needle cannulae 10. A different method to ensure that the outside surface is not exposed to the etchant 5 is to flush a non-etching liquid into the voids between the individual needle cannulae 10.

If the pressure P2 is kept at the same level as the process progresses and more and more material is etched away, the level of the etchant 5 will fall to a lower level since the volume etched away by the etchant 5 increases. This is indicated in the right half of figure 6. However, in order to compensate for the removed material the pressure P2 can be increased throughout the cycles such that the level of the etchant 5 is kept constant.

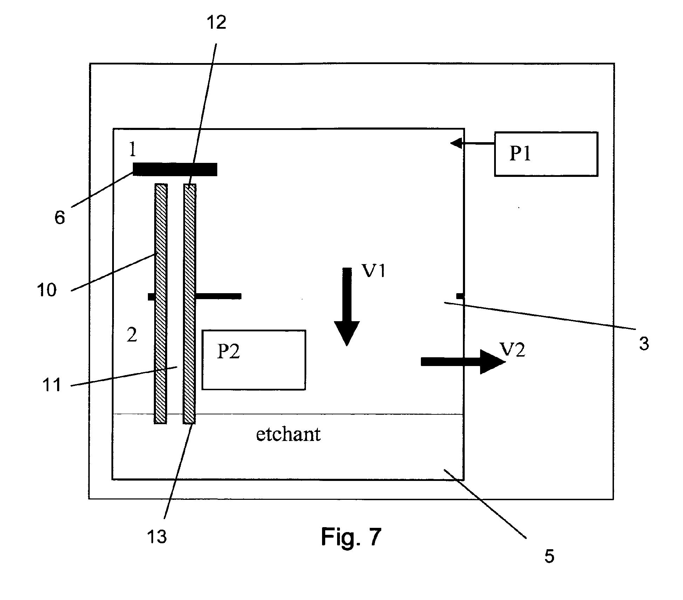

A slightly different embodiment of the method is disclosed in figure 7.

The compartment containing the first gas-chamber 1 and the second gas-chamber 2 is in this embodiment connected to each other through a first valve V1. Further, the second gas-chamber 2 is connected to the outside atmospheric pressure through a second valve V2.

The various steps of the process are now described in an example relating to figure 7. The numeric pressures including the atmospheric pressure referred to in this example can be different from the mentioned pressures. The important feature to note is that the various pressures have to be different at different steps in order to shift the etchant 5 in and out of the lumen 11.

### Step 1

P1: 1,1 bar
V1: closed
V2: open
Lid: open
Cannula: raised

At step 1, the pressure in gas-chamber 2 is the atmospheric pressure and gas flow through the lumen 11 of the needle cannulae 10 due to the slightly higher pressure inside gas-chamber 1.

### Step 2

P1: 1,1 bar
V1: closed
V2: open
Lid: open
Cannula: submerged

At step 2, the pressure in the first chamber 1 and in the lumen 11 of the needle cannula 10 is both equal to P1 and gas will continue to flow through the needle cannulae, preventing etchant 5 from entering the lumen 11.

### Step 3

P1: 1,1 bar
V1: closed
V2: open
Lid: closed
Cannula: submerged

At step 3, the lid 6 is closed and the pressure in the lumen 11 of the needle cannulae 10 becomes the same as the gas pressure inside gas-chamber 2 (P2) as any excess pressure trapped inside the lumen 11 will escape through the etchant 5.

### Step 4

P1: 5,0 bar
V1: closed
V2: open
Lid: closed
Cannula: submerged

In step 4, the pressure is being build up in the first gas-chamber 1 without entering into the lumen 11 of the needle cannulae 10 as the lid 6 is closed.

### Step 5

P1: 5,0 bar
V1: open
V2: closed
Lid: closed
Cannula: submerged

In step 5, the pressure in both gas-chamber 1 and in gas-chamber 2 reaches the same level (P1 = P2) thereby pushing the etchant 5 into the lumen 11 of the needle cannula 10. The etchant 5 flows upward inside the lumen 11 to a level where the pressure of the trapped gas inside the lumen 11 reaches P1 (= P2). In this step, the needle cannulae 10 can be removed from the bath as the pressure P2 in gas-chamber 2 maintains the etchant 5 inside the needle cannulae 10. If the pressure is released in this position, the needle cannulae 10 are emptied. This has the benefit that used etchant 5 can be removed from the bath during the process. This particular way of emptying the lumen 11 is also operational for the process according to the first embodiment.

### Step 6

P1: 1,1 bar
V1: closed
V2:open
Lid: open
Cannula: submerged

Same situation as in step 2. The pressure inside the lumen 11 equals the pressure in the gas-chamber 1.

### Step 7

P1: 1,1 bar
V1: closed
V2: open
Lid: open
Cannula: raised

Same situation as in step 1. The process is now ready to be repeated.

When the lid 6 is open and the needle cannulae 10 is not submerged, the size of the opening of the lumen 11 of the needle cannulae 11 in the process can be verified e.g. by applying a specific pressure to gas-chamber 1 and measuring the flow through the needle cannulae 10 or the pressure drop over time as further described in EP 2010-195375.

Further, in step 5 when removing the needle cannulae 10 from the bath and maintaining the etchant 5 inside the lumen 10, the needle cannulae 10 can be lowered into a new bath containing water (or another non-etching liquid) and the pressure P2 can be further increased pushing the water into the proximal end 13 of the needle cannulae 10 and moving the etchant 5 further into the lumen 11 whereby material can be removed from the centre part of the needle cannulae 10. Thus, maintaining the original inside diameter at the proximal end 13 of the needle cannulae 10.

A number of potential difficulties when carrying out the method can be envisaged. Scum can form of the surface of the etchant 5 which will make it difficult to determine the level through to which the etchant 5 will be active inside the needle cannulae 10. This can be solved by minimizing the gas flow thus keeping the creation of scum at a minimum. Bubbles can form in the etchant 5 which would potentially keep etchant 5 away from certain areas of the inside surface of the lumen 11. This can be countered by using a suitable pressure and lowering the speed by which the etchant 5 is moved in and out of the needle cannulae 10. Once the needle cannulae 10 has been removed from the etchant 5 a thin layer of etchant 5 can be present on the inside surface of the needle cannula 10 thereby preventing new etchant in obtaining contact with the inside surface of the needle cannulae 10 if the process is repeated. One way of overcoming this obstacle is to blow more gas though the needle cannulae 10 between repetitions of the process.

Some preferred embodiments have been shown in the foregoing, but it should be stressed that the invention is not limited to these, but may be embodied in other ways within the subject matter defined in the following claims. All though the method described in many aspects of the present application refer to a pen needle for injecting insulin, it is clear that the method as claimed refer to any type of needle cannula for injecting any kind of drug.

## Claims

1. A method of manufacturing needle cannulae comprising the steps of:
(i) providing a plurality of metallic needle cannulae (10) having a distal end (12) and a proximal end (13) and an oblong inside lumen (11) there between,
(ii) providing a metal etching liquid (5) surfacing a gas ,
(iii) establishing contact between the metal etching liquid (5) and one end (12, 13) of each needle cannula (10) in the plurality,
(iv) sealing off the other end (12, 13) of each needle cannula (10) in the plurality,
(v) transferring the metal etching liquid (5) into a part (14) of the oblong inside lumen (11) of the metallic needle cannula (10), by a applying pressure to the gas surfacing the metal etching liquid (5).

2. A method of manufacturing needle cannulae according to claim 1, further comprising the steps of:
(vi) releasing the applied pressure,
(vii) removing the seal (6) sealing off the opposite end (12, 13) of each needle cannulae (10).

3. A method of manufacturing needle cannulae according to claim 2 or 3, wherein the metal etching liquid (5) is contained in a bath and transferred to the lumen (11) of each needle cannulae (10) in the plurality by applying the pressure to the gas surfacing the surface of the metal etching liquid (5) in the bath.

4. A method of manufacturing needle cannulae according to claim 3, wherein the plurality of needle cannulae (10) are provided in a pressure chamber (1, 2) during the process.

5. A method of manufacturing needle cannulae according to claim 4, wherein the pressure chamber is divided into a first gas-chamber (1) and a second gas-chamber (2) by a barrier (3).

6. A method of manufacturing needle cannulae according to claim 5, wherein the lumens (11) of the pluralities of needle cannulae (10) connects the first gas-chamber (1) with the second gas-chamber (2).

7. A method of manufacturing needle cannulae according to any of the claims 2-6, wherein the steps are performed in continuous cycles.

8. A method of manufacturing needle cannulae according to any of the claims 3 to 7, further comprising the steps of
(viii) moving the proximal end (13) of the needle cannulae (10) to a second bath containing water, and
(iv) increasing the pressure of the gas to move water from the second bath into the most proximal end (13) of the needle cannulae (10).

## Patentansprüche

1. Verfahren zur Herstellung von Nadelkanülen, die folgenden Schritte umfassend:
(i) Bereitstellung einer Vielzahl von Nadelkanülen (10) mit einem distalen Ende (12) und einem proximalen Ende (13) und einem länglichen Innenlumen (11) dazwischen,
(ii) Bereitstellung einer Metallätzflüssigkeit (5) über einem Gas,
(iii) Herstellung eines Kontakts zwischen der Metallätzflüssigkeit (5) und einem Ende (12, 13) jeder Nadelkanüle (10) in der Vielzahl,
(iv) Abdichtung des anderen Endes (12, 13) jeder Nadelkanüle (10) in der Vielzahl,
(v) Überführung der Metallätzflüssigkeit (5) in einen Teil (14) des länglichen Innenlumens (11) der Metall-Nadelkanüle (10) durch Aufbringen eines Drucks auf die Gasfläche über der Metallätzflüssigkeit (5).

2. Verfahren zur Herstellung von Nadelkanülen nach Anspruch 1 ferner die folgenden Schritte umfassend:
(vi) Entspannen des aufgebrachten Drucks,
(vii) Entfernung der Dichtung (6), die das gegenüberliegende Ende (12, 13) jeder Nadelkanüle (10) abdichtet.

3. Verfahren zur Herstellung von Nadelkanülen nach Anspruch 2 oder 3, worin die Metallätzflüssigkeit (5) in einem Bad enthalten ist und zu dem Lumen (11) jeder Nadelkanüle (10) in der Vielzahl überführt wird, indem der Druck auf das Gas über der Oberfläche der Metallätzflüssigkeit (5) in dem Bad aufgebracht wird.

4. Verfahren zur Herstellung von Nadelkanülen nach Anspruch 3, worin die Vielzahl von Nadelkanülen (10) in einer Druckkammer (1, 2) während des Verfahrens bereitgestellt wird.

5. Verfahren zur Herstellung von Nadelkanülen nach Anspruch 4, worin die Druckkammer durch eine Barriere (3) in eine erste Gaskammer (1) und eine zweite Gaskammer (2) unterteilt wird.

6. Verfahren zur Herstellung von Nadelkanülen nach Anspruch 5, worin die Lumen (11) der Vielzahl von Nadelkanülen (10) die erste Gaskammer (1) mit der zweiten Gaskammer (2) verbinden.

7. Verfahren zur Herstellung von Nadelkanülen nach einem der Ansprüche 2-6, worin die Schritte in kontinuierlichen Zyklen durchgeführt werden.

8. Verfahren zur Herstellung von Nadelkanülen nach einem der Ansprüche 3 bis 7, ferner umfassend die folgenden Schritte:
(viii) Bewegen des proximalen Endes (13) der Nadelkanülen (10) in ein zweites, Wasser enthaltendes Bad, und
(iv) Erhöhung des Drucks des Gases zur Bewegung von Wasser aus dem zweiten Bad in das proximalste Ende (13) der Nadelkanülen (10).

## Revendications

1. Procédé de fabrication de canules à aiguille comprenant les étapes consistant à :
(i) fournir une pluralité de canules à aiguille métalliques (10) ayant une extrémité distale (12) et une extrémité proximale (13) et une lumière intérieure oblongue (11) entre elles ;
(ii) fournir un liquide d'attaque de métal (5) avec un gaz de surface ;
(iii) établir un contact entre le liquide d'attaque de métal (5) et une extrémité (12, 13) de chaque canule à aiguille (10) de la pluralité ;
(iv) rendre étanche l'autre extrémité (12, 13) de chaque canule à aiguille (10) de la pluralité ;
(v) transférer le liquide d'attaque de métal (5) dans une partie (14) de la lumière intérieure oblongue (11) de la canule à aiguille métallique (10) en appliquant une pression au gaz à la surface du liquide d'attaque de métal (5).

2. Procédé de fabrication de canules à aiguille selon la revendication 1, comprenant les étapes consistant à :
(vi) libérer la pression appliquée ;
(vii) ôter un joint (6) rendant étanche l'extrémité opposée (12, 13) de chaque canule à aiguille (10).

3. Procédé de fabrication de canules à aiguille selon la revendication 2 ou 3, dans lequel le liquide d'attaque de métal (5) est contenu dans un bain et transféré vers la lumière (11) de chaque canule à aiguille (10) de la pluralité en appliquant la pression au gaz à la surface du liquide d'attaque de métal (5) dans le bain.

4. Procédé de fabrication de canules à aiguille selon la revendication 3, dans lequel la pluralité de canules à aiguille (10) est fournie dans une chambre sous pression (1, 2) pendant le procédé.

5. Procédé de fabrication de canules à aiguille selon la revendication 4, dans lequel la chambre sous pression est divisée en une première chambre à gaz (1) et en une seconde chambre à gaz (2) par une barrière (3).

6. Procédé de fabrication de canules à aiguille selon la revendication 5, dans lequel les lumières (11) de la pluralité de canules à aiguille (10) connectent la première chambre à gaz (1) à la seconde chambre à gaz (2).

7. Procédé de fabrication de canules à aiguille selon l'une quelconque des revendications 2 à 6, dans lequel les étapes sont réalisées en cycles continus.

8. Procédé de fabrication de canules à aiguille selon l'une quelconque des revendications 3 à 7, comprenant les étapes consistant à :
(viii) déplacer l'extrémité proximale (13) des canules à aiguille (10) vers un second bain contenant de l'eau ; et
(iv) augmenter la pression du gaz afin de déplacer l'eau du second bain vers l'extrémité proximale (13) des canules à aiguille (10).
